# EUROPEAN PATENT SPECIFICATION

(11) **EP 1 673 072 B1**
(45) Date of publication and mention of the grant of the patent: **04.04.2007**
(21) Application number: 04817650.7
(22) Date of filing: 29.09.2004
(51) Int. Cl.: A61K 9/20

(54) **A CONTROLLED RELEASE PHARMACEUTICAL COMPOSITION AND A PROCESS FOR PREPARING THE SAME**
PHARMAZEUTISCHE ZUSAMMENSETZUNG MIT KONTROLLIERTER FREISETZUNG UND VERFAHREN ZU IHRER HERSTELLUNG
COMPOSITION PHARMACEUTIQUE A LIBERATION CONTROLEE ET PROCEDE DE PREPARATION DE CETTE COMPOSITION

(30) Priority: 01.10.2003 IN MU04172003
(43) Date of publication of application: 28.06.2006
(73) Proprietor: Lupin Limited, Mumbai 400 098, Maharashtra (IN)
(72) Inventor: SEN, Himadri, Taluka Mulshi, Pune 411 042, Maharashtra (IN); JAYANTHI, Suryakumar, Taluka Mulshi, Pune 411 042, Maharashtra (IN); RAGHAVAN, Vineeth, Taluka Mulshi, Pune 411 042, Maharashtra (IN); ARRA, Ganga, Srinivas, Taluka Mulshi, Pune 411 042, Maharashtra (IN)
(74) Representative: Harrison Goddard Foote
(86) International application number: PCT/IN2004/000306
(87) International publication number: WO 2005/048978

(56) References cited:
- WO-A-00/18383
- US-B1- 6 177 435
- DATABASE WPI Section Ch, Week 200275 Derwent Publications Ltd., London, GB; Class B02, AN 2002-699216 XP002357486 & ZA 200 110 500 A (CIPLA MEDPRO PTY LTD) 29 May 2002 (2002-05-29)

## Description

### FIELD OF THE INVENTION

The present invention relates to antiretroviral pharmaceutical composition. The invention particularly relates to an antiretroviral pharmaceutical composition having a selective combination of a controlled release active formulation and an immediate release active formulation for once daily administration. The invention also relates to the process for manufacture of such once daily antiretroviral pharmaceutical composition.

### BACKGROUND OF THE INVENTION

Acquired Immune Deficiency Syndrome (AIDS) which is caused by the human immunodeficiency virus (HIV) is one of the few diseases for which mankind is struggling to find a cure.

In the last several years many antiretroviral agents have been discovered that have since been used to treat AIDS. The drugs that are currently approved for anti-HIV therapy are broadly classified into three categories, namely:
1. Nucleoside Reverse Transcriptase Inhibitors (NRTI), which include lamivudine, zidovudine, didanosine, abacavir, stavudine, and zalcitabine.
2. Non-nucleoside Reverse Transcriptase Inhibitors (NNRTI), which include nevirapine, efavirenz, and delavirdine.
3. Protease Inhibitors (PI), which include indinavir, ritonavir, nelfinavir, saquinavir and amprenavir.

For successful treatment of any disease caused by a microorganism the drug used in the therapy should eliminate the causative organism completely without allowing them to undergo mutation. Mutation may lead to resistant strains that can make treatment more difficult. This development of resistant strains is usually observed when a single agent or drugs belonging to a single category are solely used in the treatment. For most diseases caused by microorganisms, it has been found that combining two or more drugs, preferably from different classes have resulted in a greater success rate.

Drugs for the treatment of highly active antiretroviral therapy were initially prescribed as a loose combination of two or three drugs. This was rationalised to fixed dose combinations to be administered twice daily. In our co-pending application number PCT/IN02/00110 we have described a formulation, which further reduced the pill burden to once a day. However to date there are no reports of once a day formulation comprising a three drug combination.

There are three known alternative regimens as follows:
1) 3 NRTIs
2) 2 NRTIs + 1 NNRTI
3) 2 NRTIs + 1 PI

Nucleoside-based reverse transcriptase inhibitors (NRTIs) were the first drugs to be given as antiretroviral agents, and they remain the backbone of therapy against HIV infection and acquired immuno deficiency syndrome (AIDS).

Although various therapy regimens have been tried as indicated above, it has been reported that drug combination comprising three NRTIs is not as effective as other combinations. The combination of two NRTIs, lamivudine and zidovudine which form the backbone of therapy with one NNRTI such as Nevaripine or Efavirenz is ideal. The serum half-life of Lamivudine is about 3 to 6 hours while that of Zidovudine is 1.1 hours. Nevaripine has a long half-life of more than 25 hours and is currently given as 200 mg twice daily. Efavirenz is approved for a dosing of 600 mg once daily.

We propose to use 400 mg Nevaripine to be given as a fixed dose combination with lamivudine and zidovudine once daily. In case of Efavirenz the amount of efavirenz or pharmaceutically acceptable derivative thereof is 600-800mg preferably.

Multi-drug therapy in case of antiretrovirals has the advantage that drugs have different mechanisms of action and act at different stages of the viral life cycle and they may exhibit a synergistic effect on such use. However, this kind of therapy results in the patient having to take multiple pills several times a day and this is known to cause problems of compliance in following the therapy regimen. Several attempts have been made to reduce the pill burden in order to enhance patient compliance.

Lamivudine 150mg and zidovudine 300mg are NRTIs that are to be given twice daily. A fixed dose combination of lamivudine 150mg and zidovudine 300mg (COMBIVIR) has been developed which has reduced the pill load for this combination to two. Similarly, a three drug fixed dose combination of lamivudine 150 mg, zidovudine 300 mg and abacavir 300 mg (TRIZIVIR) has also been developed. However, there are reports of toxicity related to abacavir. Further, the frequency of dosing for both two and three drug combination is still twice daily.

United States Patent No. 6,113,920 discloses a pharmaceutical composition comprising two active pharmaceutical ingredients namely lamivudine and zidovudine and a pharmaceutically acceptable glidant ingredient, selected from a group of colloidal silicon dioxide, microcrystalline cellulose, metallic stearates, calcium carbonate and combinations thereof, in the form of a film coated tablet. The composition contains lamivudine in an amount from 15 to 1500 mg per tablet and zidovudine in an amount from 30 to 1000 mg per tablet. This combination is given twice daily.

United States Patent No. 4,917,900 discloses a pharmaceutical formulation for oral administration in which discrete units comprising zidovudine are provided with a controlled release coating consisting of alkyl esters of acrylic and methacrylic acids and ethylcellulose in a weight ratio of 1:3 to 3:1. These spheroids contain at least 80% of zidovudine and microcrystalline cellulose and mannitol as the core-forming agent.

As discussed above, while a two drug combination product may reduce the pill burden to half, it still has to be given twice daily and when coupled with a third drug, the pill burden is substantially increased.

### OBJECTS OF THE INVENTION

It is thus the basic object of the present invention to provide for a three-drug antiretroviral pharmaceutical composition, which would reduce the pill load and frequency of drug administration thereby favouring patient compliance and effective treatment.

Another object is to provide a fixed dose combination of lamivudine, zidovudine and atleast one NNRTI drug suitable for once daily administration, which would reduce the pill burden to one and the frequency to once daily.

Yet further object of the present invention is directed to the development of a three drug fixed dose combination comprising essentially the antiretroviral drugs lamivudine, zidovudine, and at least one NNRTI drug preferably selected from nevirapine and efavirenz suitable for once daily dosing wherein lamivudine and zidovudine would have a controlled release while nevirapine/ efavirenz will be immediately released.

Yet another object of the present invention is related to a method of increasing the in vivo half life of Lamivudine and Zidovudine while not affecting the half life of Nevirapine or Efavirenz and thus reducing the pill burden in a patient suffering from HIV infection and / or Acquired Immunodefficiency Syndrome by administering a three drug antiretroviral composition which comprises of Lamivudine and Zidovudine as a controlled release component and nevirapine or efavirenz as an immediate release component

Yet further object is directed to provide a process for preparing an antiretroviral pharmaceutical composition as above which would reduce the pill load and frequency of drug administration thereby favouring patient compliance and effective treatment.

### SUMMARY OF THE INVENTION

Thus according to the present invention there is provided an antiretroviral pharmaceutical composition comprising a selective combination of
i. a controlled release formulation comprising :
   a. lamivudine or a pharmaceutically acceptable derivative thereof,
   b. zidovudine or a pharmaceutically acceptable derivative thereof, and
   c. a mixture of hydrophilic polymers, said polymers being selected from the group consisting of cellulose ethers, polyuronic acids and pharmaceutically acceptable gums or mixtures thereof and
   d. a pharmaceutically acceptable calcium salt.
ii. an immediate release formulation comprising at least one selective non-nucleoside reverse transcriptase inhibitors (NNRTI) drug or a pharmaceutically acceptable derivative thereof along with pharmaceutically acceptable excipients.

In accordance with another aspect of the present invention there is provided a process for the preparation of a pharmaceutical composition comprising
(i) mixing together active ingredients selected from amongst lamivudine, zidovudine or pharmaceutically acceptable derivatives thereof or mixtures thereof with hydrophilic polymers selected from amongst cellulose ethers, polyuronic acids, pharmaceutically acceptable gums or mixtures thereof, and with a pharmaceutically acceptable calcium salt, optionally a diluent and a lubricant to provide a controlled release formulation
(ii) providing an immediate release formulation obtained of at least one NNRTI or pharmaceutically acceptable derivatives thereof blended with pharmaceutically acceptable excipients and
(iii) obtaining a composition therefrom by compressing the resulting blends into bilayered tablets, or by applying the said immediate release formulation as an outer coat over the core of the said controlled release formulation

### DETAILED DESCRIPTION OF THE INVENTION

The pharmaceutical composition is preferably in the form of a bilayered tablet with the controlled release comprising one layer and the immediate release comprising the second layer. Alternatively the controlled release component may be in the form of a core and the second immediate release layer may be coated on top of the core.

The controlled release layer of the pharmaceutical composition comprises lamivudine and zidovudine or their pharmaceutically acceptable derivatives along with a mixture of hydrophilic polymers selected from the group consisting of cellulose ethers, polyuronic acids, pharmaceutically acceptable gums, or mixtures thereof.

In addition, this layer also comprises a pharmaceutically acceptable calcium salt and optionally one or more water-soluble or water dispersible pharmaceutically acceptable excipients.

The cellulose ether used in accordance with the present invention is selected from amongst those commonly known in the art such as hydroxypropyl cellulose, hydoxypropyl methylcellulose, carboxy methylcellulose, sodium carboxymethylcellulose, ethyl cellulose, methyl cellulose, hydroxy ethyl cellulose and the like. It is present in an amount from about 2% to about 12% by weight of the controlled release layer and preferably in an amount from about 3% to about 8% by weight of the controlled release layer.

The polyuronic acid used in accordance with the present invention is selected from amongst alginic acid, sodium alginate, calcium alginate, sodium calcium alginate, potassium alginate, ammonium alginate, magnesium alginate and the like. It is present in an amount from about 0.5% to about 10% by weight of the controlled release layer and preferably in an amount from about 1% to about 6% by weight of the controlled release layer.

The pharmaceutically acceptable gum used in the composition of the invention is selected from amongst those commonly known in the art such as guar gum, xanthan gum, gum karaya, tragacanth gum, gum acacia and the like. It is present in an amount from about 0.1 % to about 10% by weight of the controlled release layer and preferably in an amount from about 0.5% to about 6% by weight of the controlled release layer.

Calcium salts when used along with certain polymers, especially the alginates, have been known to stabilize the matrix. In accordance with this, in a preferred embodiment, the said controlled release layer of the composition also contains a pharmaceutically acceptable calcium salt.

The calcium salt is selected from the group consisting of calcium sulphate, calcium phosphate, calcium carbonate and calcium chloride. It is present in an amount from about 0.1 % to about 2.5% by weight of the controlled release layer preferably from about 0.1 % to about 2% by weight of the controlled release layer.

The controlled release layer of the composition may further contain one or more pharmaceutically acceptable other excipients selected from amongst water-soluble and/or water dispersible diluents and lubricants.

The water dispersible or water soluble diluent selected from amongst microcrystalline cellulose, dicalcium phosphate, calcium carbonate, lactose, powdered cellulose, starch, mannitol and the like. They are present from about 1% to about 28% by weight of the controlled release layer.

When the diluent is microcrystalline cellulose,it is present in an amount from about 5% to about 20% by weight of the controlled release layer.

When the diluent is dicalcium phosphate, it is present in an amount from about 1% to about 5% by weight of the controlled release layer.

The immediate release layer comprises at least one NNRTI selected from amongst nevirapine and efavirenz or their pharmaceutically acceptable derivatives

The immediate release layer may further comprise pharmaceutically acceptable excipients selected from the group consisting of diluents, binders, disintegrants, lubricants, coloring agents arid the like.

The diluent is selected from amongst microcrystalline cellulose, dicalcium phosphate, calcium carbonate, lactose, powdered cellulose, starch, mannitol and the like. The diluent is present in an amount from about 2% to about 15% by weight of the immediate release layer.

The binder is selected from amongst carboxymethylcellulose sodium, povidone, pregelatinised starch, gelatin, and the like. The binder is present in an amount from about 1% to about 10% by weight of the immediate release layer.

The disintegrant is selected from amongst crospovidone, sodium starch glycolate, pregelatinised starch, carboxymethylcellulose sodium, croscarmellose sodium, starch and the like. It is present in an amount from about 0.5% to about 15% by weight of the immediate release layer.

Each layer may also contain lubricants selected from amongst those commonly known in the art such as magnesium stearate, calcium stearate, stearic acid, silicon dioxide, talc and the like. It is present in an amount from about 0.1% to about 3% by weight of each layer.

The pharmaceutical compositions according to the invention may be prepared by procedures well known to those skilled in the art. According to a convenient method for the preparation of the granular blend for each layer, all the active ingredients along with the necessary excipients are mixed together and then compacted. The compacted mass is then comminuted to obtain the granules. Alternatively, the granules may also be prepared by the process of wet granulation using a suitable granulating agent.

The final granular blend of the two layers are either compressed into bilayered tablets on a compression machine suitable for such purpose or the controlled release layer may be present as a core and the immediate release layer is present as a coat around the core. The tablets so obtained may be further coated using a water-soluble polymer.

The above-mentioned process results in a pharmaceutical composition that contains three antiretroviral agents in one tablet suitable for once daily administration. The effective therapeutic dose of the active that can be administered include a combination of 300 mg of lamivudine, 600 mg of zidovudine and 400 mg of nevirapine/ 600 mg of efavirenz.

The composition of the invention and its advantages are explained hereunder in greater detail in relation to examples hereunder:

### Example 1

| **Ingredients** | **Weight (mg/tab)** |
|---|---|
| **Controlled Release Layer** | |
| Lamivudine | 300.0 |
| Zidovudine | 600.0 |
| Microcrystalline Cellulose | 187.0 |
| Hydroxypropyl methylcellulose | 62.5 |
| Sodium alginate | 31.25 |
| Guar gum | 12.5 |
| Calcium sulphate | 3.75 |
| Dicalcium phosphate | 40.0 |
| Magnesium Stearate | 13.0 |

| **Immediate Release Layer** | |
|---|---|
| Nevirapine | 400.0 |
| Powdered Cellulose | 52.5 |
| Povidone K30 | 20.0 |
| Sodium starch glycolate | 15.0 |
| Magnesium stearate | 3.75 |
| Sodium starch glycolate | 5.0 |
| Colloidal silicon dioxide | 2.5 |
| Magnesium stearate | 1.25 |

Controlled release layer blend: Lamivudine, zidovudine, hydrophilic polymers, calcium sulphate, dicalcium phosphate, and microcrystalline cellulose were screened through 30 no. mesh and mixed with magnesium stearate. The blend was compacted and the slugs obtained were milled to form granules. The sized granules were blended with fines and lubricated.

**Immediate release layer blend:** Nevirapine, cellulose, povidone and a first portion of sodium starch glycolate were screened through 40 no. mesh and mixed with magnesium stearate. The blend was compacted and the slugs obtained were milled to form granules. The granules were mixed with a second portion of sodium starch glycolate, colloidal silicon dioxide and lubricated.

**Bilayered tablets:** Both the above blends were compressed into bilayered tablets using a bilayered tablet compression machine.

**Drug Release:** The tablets were tested for release of all three actives using USP Type 1 Dissolution apparatus at an RPM of 100 and with 900ml of 0.1N HCL for the first 2 hrs and pH 6.8 Phosphate buffer afterwards.

| **Time** | **% Drug released** | | |
|---|---|---|---|
| | Lamivudine | Zidovudine | Nevirapine |
| 1hr | 29.1 | 17.1 | 87.1 |
| 2hrs | 42.7 | 25.9 | |
| 4hrs | 55.5 | 42.3 | |
| 8hrs | 70.4 | 67.6 | |
| 10hrs | 79.4 | 80.1 | |
| 12hrs | 85.3 | 88.2 | |

### Example 2

| **Ingredients** | **Weight (mg/tab)** |
|---|---|
| **Controlled Release Layer** | |
| Lamivudine | 300.0 |
| Zidovudine | 600.0 |
| Microcrystalline Cellulose | 187.0 |
| Hydroxypropyl methylcellulose | 62.5 |
| Sodium alginate | 31.25 |
| Guar gum | 12.5 |
| Calcium sulphate | 3.75 |
| Dicalcium phosphate | 40.0 |
| Magnesium Stearate | 13.0 |

| **Immediate Release Layer** | |
|---|---|
| Nevirapine | 400.0 |
| Powdered Cellulose | 35.0 |
| Povidone K30 | 20.0 |
| Crospovidone . | 15.0 |
| Sodium starch glycolate | 20.0 |
| Sunset Yellow FCF | 2.5 |
| Magnesium stearate | 5.0 |
| Colloidal silicon dioxide | 2.5 |

The manufacturing procedure of Example 1 was followed.

The release of the drugs was obtained as follows:

| **Time** | **% Drug released** | | |
|---|---|---|---|
| | Lamivudine | Zidovudine | Nevirapine |
| 1hr | - | - . | 94.2 |
| 2hrs | 47.0 | 26.8 | |
| 4hrs | 61.7 | 43.0 | |
| 8hrs | 71.3 | 64.8 | |
| 10hrs | 79.1 | 75.4 | |
| 12hrs | 86.6 | 76.7 | |
| 14hrs | 90.5 | 89.2 | |

### Example 3

| **Ingredients** | **Weight (mg/tab)** |
|---|---|
| **Controlled Release layer** | |
| Lamivudine | 300.0 |
| Zidovudine | 600.0 |
| Microcrystalline Cellulose | 187.0 |
| Hydroxypropyl methylcellulose | 62.5 |
| Sodium alginate | 31.25 |
| Guar gum | 12.5 |
| Calcium sulphate | 3.75 |
| Dicalcium phosphate | 40.0 |
| Magnesium Stearate | 13.0 |

| **Immediate Release layer** | |
|---|---|
| Nevirapine | 400.0 |
| Powdered Cellulose | 42.5 |
| Povidone K30 | 10.0. |
| Crospovidone | 20.0 |
| Sodium starch glycolate | 15.0 |
| Magnesium stearate | 3.75 |
| Sodium starch glycolate | 5.0 |
| Colloidal silicon dioxide | 2.5 |
| Magnesium stearate | 1.25 |

The manufacturing procedure of Example 1 was followed

The release obtained of the drugs is as follows:

| **Time** | **% Drug released** | | |
|---|---|---|---|
| | Lamivudine | Zidovudine | Nevirapine |
| 1hr | 33.8 | 21.3 | 97.1 |
| 2hrs | 50.5 | 31.0 | |
| 4hrs | 67.8 | 49.5 | |
| 8hrs | 79.2 | 74.0 | |
| 10hrs | 86.2 | 83.7 | |
| 12hrs | 90.6 | 90.1 | |

### Example 4

| **Ingredients** | **Weight (mg/tab)** |
|---|---|
| **Core** | |
| Lamivudine | 300.0 |
| Zidovudine | 600.0 |
| Microcrystalline Cellulose | 187.0 |
| Hydroxypropyl methylcellulose | 62.5 |
| Sodium alginate | 31.25 |
| Guar gum | 12.5 |
| Calcium sulphate | 3.75 |
| Dicalcium phosphate | 40.0 |
| Magnesium Stearate | 13.0 |

| **Coat** | |
|---|---|
| Nevirapine | 400.0 |
| Powdered Cellulose | 32.5 |
| Povidone K30 | 20.0 |
| Crospovidone | 20.0 |
| Sodium starch glycolate | 15.0 |
| Magnesium stearate | 3.75 |
| Sodium starch glycolate | 5.0 |
| Colloidal silicon dioxide | 2.5 |
| Magnesium stearate | 1.25 |
| Sunset Yellow FCF | 2.5 |

**Core:** Lamivudine, zidovudine, hydrophilic polymers, calcium sulphate, dicalcium phosphate, and microcrystalline cellulose were screened through 30 no. mesh and mixed with magnesium stearate. The blend was compacted and the slugs obtained were milled to form granules. The sized granules were blended with fines, lubricated and compressed into a tablet.

Coat: Nevirapine, cellulose, povidone and a first portion of sodium starch glycollate were screened through 40 no. mesh and mixed with magnesium stearate. The blend was compacted and the slugs obtained were milled to form granules. The granules were mixed with a second portion of sodium starch glycolate, colloidal silicon dioxide, lubricated and compression coated over the core tablet.

The release obtained of the drugs is as follows:

| **Time** | **% Drug released** | | |
|---|---|---|---|
| | Lamivudine | Zidovudine | Nevirapine |
| 1hr | 37.4 | 22.8 | 80.8 |
| 2hrs | 54.1 | 32.1 | |
| 4hrs | 66.5 | 47.5 | |
| 8hrs | 82.5 | 76.8 | |
| 10hrs | 92 | 87.3 | |
| 12hrs | 97.3 | 93.7 | |

A single dose fed study was conducted using the tablets of Example 1 against a commercially available immediate release combination of the three drugs. The results of the study are tabulated in Table 1.

| | Test: Lamivudine 300 mg (as ER) + Zidovudine 600mg (as ER) + Nevirapine 400mg (OD) tablets | | | Reference: Lamivudine 150 mg +Zidovudine 300 mg + Nevirapine 200 mg (IR/BD) tablets | | |
|---|---|---|---|---|---|---|
| | Lamivudine | Zidovudine | Nevirapine | Lamivudine | Zidovudine | Nevirapine |
| Cₘₐₓ (mcg/ml) | 1.79 | 1.54 | 5.17 | 1.51 | 1.49 | 2.88 |
| AUC (0-t) (mcg.hr/ml) | 12.18 | 7.45 | 36.37 | 5.84 | 3.12 | 18.55 |
| t_{1/2} (hr) | 5.67 | 2.74 | 29.34 | 2.98 | 0.72 | 17.82 |

The results indicate that the composition of the present invention achieves a Cₘₐₓ of Lamivudine and Zidovudine, which is equivalent to that obtained when the same drugs are administered in immediate release form. However, the invention also succeeds in increasing the AUC₀₋ₜ values and the half lives of all the drugs in vivo.

It is thus apparent from the above exemplary illustrations that the pharmaceutical composition of the invention serves as a three-drug antiretroviral combination for once daily dosage for a combination treatment especially of NRTIs and NNRTIs. The once daily dosage form of the invention is simple and cost-effective and would serve in reducing the pill burden and frequency of administration and favour patient compliance with the desired drug regime for effective treatment.

## Claims

1. An antiretroviral pharmaceutical composition comprising a selective combination of
i. a controlled release formulation comprising:
a. lamivudine or a pharmaceutically acceptable derivative thereof,
b. zidovudine or a pharmaceutically acceptable derivative thereof, and
c. a mixture of hydrophilic polymers, said polymers being selected from the group consisting of cellulose ethers, polyuronic acids and pharmaceutically acceptable gums or mixtures thereof and
d. a pharmaceutically acceptable calcium salt.
ii. an immediate release formulation comprising atleast one selective Non-nucleoside Reverse Transcriptase Inhibitors (NNRTI) drug or a pharmaceutically acceptable derivative thereof along with pharmaceutically acceptable excipients.

2. An antiretroviral pharmaceutical composition as claimed in claim 1 wherein said NNRTI drug is selected from nevirapine, and efavirenz.

3. An antiretroviral pharmaceutical composition as claimed in claim 1 which is in the form of two separate layers of said controlled release and the immediate release formulation

4. An antiretroviral pharmaceutical composition as claimed in claim 1 which is in the form of a core of said controlled release formulation and an outer coat of the immediate release formulation.

5. An antiretroviral pharmaceutical composition as claimed in claim 1 comprising a bilayer selective combination of
i. a first layer of controlled release formulation comprising :
a. lamivudine or a pharmaceutically acceptable derivative thereof,
b. zidovudine or a pharmaceutically acceptable derivative thereof, and
c. a mixture of hydrophilic polymers, said polymers being selected from the group consisting of cellulose ethers, polyuronic acids and pharmaceutically acceptable gums or mixtures thereof and
d. a pharmaceutically acceptable calcium salt.
ii. a second layer of an immediate release formulation wherein said NNRTI is nevirapine or a pharmaceutically acceptable derivative thereof along with pharmaceutically acceptable excipients.

6. An antiretroviral pharmaceutical composition as claimed in claim 1 comprising a selective combination of
i. a core having a controlled release formulation comprising:
a. lamivudine or a pharmaceutically acceptable derivative thereof,
b. zidovudine or a pharmaceutically acceptable derivative thereof, and
c. a mixture of hydrophilic polymers, said polymers being selected from the group consisting of cellulose ethers, polyuronic acids and pharmaceutically acceptable gums or mixtures thereof and
d. a pharmaceutically acceptable calcium salt.
ii. an outer coat of an immediate release formulation wherein said NNRTI is nevirapine or a pharmaceutically acceptable derivative thereof and pharmaceutically acceptable excipients.

7. An antiretroviral pharmaceutical composition as claimed in claim 1 comprising a selective bilayer combination of
i. a first layer of a controlled release formulation comprising:
a. lamivudine or a pharmaceutically acceptable derivative thereof,
b. zidovudine or a pharmaceutically acceptable derivative thereof, and
c. a mixture of hydrophilic polymers, said polymers being selected from the group consisting of cellulose ethers, polyuronic acids and pharmaceutically acceptable gums or mixtures thereof and
d. a pharmaceutically acceptable calcium salt.
ii. a second layer of an immediate release formulation wherein said NNRTI is efavirenz or a pharmaceutically acceptable derivative thereof along with pharmaceutically acceptable excipients.

8. An antiretroviral pharmaceutical composition as claimed in claim 1 comprising a selective combination of
i. a core of controlled release formulation comprising :
a. lamivudine or a pharmaceutically acceptable derivative thereof,
b. zidovudine or a pharmaceutically acceptable derivative thereof, and
c. a mixture of hydrophilic polymers, said polymers being selected from the group consisting of cellulose ethers, polyuronic acids and pharmaceutically acceptable gums or mixtures thereof and
d. a pharmaceutically acceptable calcium salt.
ii. an outer coat of an immediate release formulation wherein said NNRTI is efavirenz or a pharmaceutically acceptable derivative thereof along with pharmaceutically acceptable excipients.

9. The composition as claimed in anyone of claims 1 to 8 wherein the amount of lamivudine or pharmaceutically acceptable derivative thereof is from 50 mg to 500 mg.

10. The composition as claimed in claim 9 wherein the amount of lamivudine or pharmaceutically acceptable derivative thereof is 300 mg.

11. The composition as claimed in anyone of claims 1 to 8 wherein the amount of zidovudine or pharmaceutically acceptable derivative thereof is from 100 mg to 1000 mg.

12. The composition as claimed in claim 11 wherein the amount of zidovudine or pharmaceutically acceptable derivative thereof is 600 mg.

13. The composition as claimed in anyone of claims 1 to 12 wherein the amount of nevirapine /efavirenz or pharmaceutically acceptable derivative thereof is from 100 mg to 1000 mg.

14. The composition as claimed in claim 13 wherein the amount of nevirapine or pharmaceutically acceptable derivative thereof is 400 mg.

15. The composition as claimed in claim 13 wherein the amount of efavirenz or pharmaceutically acceptable derivative thereof is 600 mg.

16. A composition as claimed in anyone of claims 1 to 15 wherein the cellulose ether is selected from amongst hydroxypropyl cellulose, hydoxypropyl methylcellulose, carboxy methylcellulose, sodium carboxymethylcellulose, ethyl cellulose, methyl cellulose, hydroxy ethyl cellulose and the like.

17. A composition as claimed in claim 16 wherein the cellulose ether is hydroxypropyl methylcellulose and is present in an amount from 2% to 12% by weight of the controlled release formulation.

18. A composition as claimed in claim 17 wherein the cellulose ether is hydroxypropyl methylcellulose and is present in an amount from 3% to 8% by weight of the controlled release formulation.

19. A composition as claimed in anyone of claims 1 to 18 wherein the polyuronic acid is selected from amongst alginic acid, sodium alginate, calcium alginate, sodium calcium alginate, potassium alginate, ammonium alginate, magnesium alginate and the like.

20. A composition as claimed in claim 19 wherein the polyuronic acid is sodium alginate and is present in an amount from 0.5% to 10% by weight of the controlled release formulation.

21. A composition as claimed in claim 20 wherein the polyuronic acid is sodium alginate and is present in an amount from 1% to 6% by weight of the controlled release formulation.

22. A composition as claimed in anyone of claims 1 to 21 wherein the pharmaceutically acceptable gum is selected from amongst guar gum, xanthan gum, gum karaya, tragacanth gum, gum acacia and the like.

23. A composition as claimed in claim 22 wherein the pharmaceutically acceptable gum is guar gum and is present in an amount from 0.1% to 10% by weight of the controlled release formulation.

24. A composition as claimed in claim 23 wherein the pharmaceutically acceptable gum is guar gum and is present in an amount from 0.5% to 6% by weight of the controlled release formulation.

25. The composition as claimed in anyone of claims 1 to 24 wherein the pharmaceutically acceptable calcium salt is selected from the group consisting of calcium sulphate, calcium phosphate, calcium carbonate and calcium chloride.

26. A composition as claimed in claim 25 wherein the pharmaceutically acceptable calcium salt is calcium sulphate and is present in an amount from 0.1% to 2.5% by weight of the controlled release formulation.

27. A composition as claimed in claim 26 wherein the pharmaceutically acceptable calcium salt is calcium sulphate and is present in an amount from 0.1 % to 2% by weight of the controlled release formulation.

28. The composition as claimed in anyone of claims 1 to 27 wherein the controlled release formulation further comprises at least one water dispersible or water soluble diluent selected from amongst microcrystalline cellulose, dicalcium phosphate, calcium carbonate, lactose, powdered cellulose, starch, mannitol and the like.

29. The composition as claimed in claim 28 wherein the diluent is present from 1% to 28% by weight of the controlled release formulation

30. The composition as claimed in claim 29 wherein the diluent is microcrystalline cellulose.

31. The composition as claimed in claim 30 wherein the amount of microcrystalline cellulose is from 5% to 20% by weight.

32. The composition as claimed in claim 29 wherein the diluent is dicalcium phosphate.

33. The composition as claimed in claim 32 wherein the amount of dicalcium phosphate is from 1% to 5% by weight.

34. The composition as claimed in anyone of claims 1 to 33 wherein the controlled release formulation further comprises at least one lubricant selected from amongst magnesium stearate, calcium stearate, stearic acid, silicon dioxide, talc and the like.

35. The composition as claimed in claim 34 wherein the lubricant is present from 0.1% -3% by weight.

36. The composition as claimed in anyone of claims 1 to 35 wherein the immediate release formulation comprises from 10% to 95% by weight of nevirapine or a pharmaceutically acceptable derivative thereof along with one or more pharmaceutically acceptable excipients selected from amongst diluents, binders, disintegrants, lubricants, coloring agents and the like.

37. A composition as claimed in claim 36 wherein the diluent is selected from amongst microcrystalline cellulose, dicalcium phosphate, calcium carbonate, lactose, powdered cellulose, starch, mannitol and the like.

38. A composition as claimed in claim 37 wherein the diluent is powdered cellulose and is present in an amount from 2% to 15% by weight of the immediate release formulation

39. A composition as claimed in claim 36 wherein the binder is selected from amongst carboxymethylcellulose sodium, povidone, pregelatinised starch, gelatin or mixtures thereof.

40. A composition as claimed in claim 39 wherein the binder is present in an amount from 1% to 10% by weight of the immediate release formulation

41. A composition as claimed in claim 36 wherein the disintegrant is selected from amongst crospovidone, sodium starch glycolate, pregelatinised starch, carboxymethylcellulose sodium, croscarmellose sodium, starch and mixtures thereof.

42. A composition as claimed in claim 41 wherein the disintegrant is present in an amount from 0.5% to 15% by weight of the immediate release formulation.

43. The composition as claimed in claim 36 wherein the lubricant is selected from amongst magnesium stearate, calcium stearate, stearic acid, silicon dioxide, talc and the like.

44. The composition as claimed in claim 43 wherein the lubricant is present from 0.1% -3% by weight.

45. A process for the preparation of an antiretroviral pharmaceutical composition comprising (i) providing a controlled release formulation by mixing together active ingredients selected from amongst lamivudine, zidovudine or mixtures thereof with hydrophilic polymers selected from amongst cellulose ethers, polyuronic acids, pharmaceutically acceptable gums or mixtures thereof, and with a pharmaceutically acceptable calcium salt, optionally a diluent and a lubricant, (ii) blending an immediate release formulation obtained of at least the NNRTI is blended with pharmaceutically acceptable excipients and (iii) obtaining the composition therefrom by compressing the resulting blends into bilayered tablets.

46. A process for the preparation of an antiretroviral pharmaceutical composition comprising (i) providing a core of said controlled release formulation by mixing together active ingredients selected from amongst lamivudine, zidovudine or mixtures thereof with hydrophilic polymers selected from amongst cellulose ethers, polyuronic acids, pharmaceutically acceptable gums or mixtures thereof, and with a pharmaceutically acceptable calcium salt, optionally a diluent and a lubricant, (ii) providing an outer coat of an immediate release formulation obtained of at least the NNRTI selected from Nevirapine, and Efavirenz blended with pharmaceutically acceptable excipients and (iii) obtaining the composition therefrom by applying the said outer coat over the said core by compression coating.

47. A process as described in claim 45 or 46 wherein each blend is dry granulated prior to compression.

48. A process as described in claim 45 or 46 wherein each blend is wet granulated prior to compression.

49. Use of three drug antiretroviral pharmaceutical composition comprising a combination of lamivudine and zidovudine as a controlled release component and nevirapine or efavirenz as an immediate release component for preparation of medicament for reducing the pill burden in a patient suffering from HIV infection and /or Acquired Immunodeficiency Syndrome.

50. Use of three drug antiretroviral pharmaceutical composition comprising a combination of lamivudine and zidovudine as a controlled release component and nevirapine or efavirenz as an immediate release component for preparation of medicament for reducing the pill burden in a patient suffering from HIV infection and / or Acquired Immunodeficiency Syndrome such that the composition upon administration to the said patient provides a Cₘₐₓ of about 1.2 to 2.0 mcg / ml for Lamivudine, about 1.0 to 2.0 mcg / ml for Zidovudine and about 4.5 to 5.5 mcg /ml for Nevirapine.

51. Use of three drug antiretroviral pharmaceutical composition comprising a combination of lamivudine and zidovudine as a controlled release component and nevirapine or efavirenz as an immediate release component for preparation of medicament for reducing the pill burden in a patient suffering from HIV infection and / or Acquired Immunodeficiency Syndrome such that the composition upon administration to the said patient provides a AUC₀₋ₜ of about 8 to 14 mcg.hr / ml for Lamivudine, about 5 to 9 mcg.hr / ml for Zidovudine and about 32 to 40 mcg.hr / ml for Nevirapine.

52. Use of three drug antiretroviral pharmaceutical composition comprising a combination of lamivudine and zidovudine as a controlled release component and nevirapine or efavirenz as an immediate release component for preparation of medicament for increasing the in vivo half life of Lamivudine and Zidovudine while not affecting the half life of Nevirapine and thus reducing the pill burden in a patient suffering from HIV infection and / or Acquired Immunodeficiency Syndrome.

53. The use of Claim 51 wherein the said composition upon administration to the said patient provides a t_{1/2} of about 5 hrs for Lamivudine, about 2 hrs for Zidovudine and about 29 hrs for Nevirapine.

54. Use of three drug antiretroviral pharmaceutical composition comprising a combination of lamivudine and zidovudine as a controlled release component and nevirapine or efavirenz as an immediate release component for preparation of medicament for reducing the pill burden in a patient suffering from HIV infection and / or Acquired Immunodeficiency Syndrome such that the composition upon administration to the said patient provides a Cₘₐₓ of the said drugs in the controlled release component which is substantially similar to that provided by immediate release compositions of the said drugs when taken simultaneously, sequentially or concurrently.

## Patentansprüche

1. Antiretrovirales Arzneimittel, umfassend eine selektive Kombination aus
i. einer Formulierung zur kontrollierten Freisetzung, umfassend:
a. Lamivudin oder ein pharmazeutisch verträgliches Derivat davon,
b. Zidovudin oder ein pharmazeutisch verträgliches Derivat davon und
c. ein Gemisch aus hydrophilen Polymeren, wobei die Polymere aus Celluloseethern, Polyuronsäuren und pharmazeutisch verträglichen Gummis oder Gemischen davon ausgewählt sind, und
d. ein pharmazeutisch verträgliches Calciumsalz.
ii. einer Formulierung zur sofortigen Freisetzung, umfassend mindestens einen selektiven Nichtnukleosid-Reverse-Transkriptase-Inhibitoren-(NNRTI) Arzneistoff oder ein pharmazeutisch verträgliches Derivat davon zusammen mit pharmazeutisch verträglichen Excipienten.

2. Antiretrovirales Arzneimittel nach Anspruch 1, wobei der NNRTI-Arzneistoff aus Nevirapin und Efavirenz ausgewählt ist.

3. Antiretrovirales Arzneimittel nach Anspruch 1, das in Form von zwei getrennten Schichten aus der Formulierung zur kontrollierten Freisetzung und der zur sofortigen Freisetzung vorliegt.

4. Antiretrovirales Arzneimittel nach Anspruch 1, das in Form eines Kerns aus der Formulierung zur kontrollierten Freisetzung und einer äußeren Beschichtung aus der Formulierung zur sofortigen Freisetzung vorliegt.

5. Antiretrovirales Arzneimittel nach Anspruch 1, umfassend eine selektive doppelschichtige Kombination aus
i. einer ersten Schicht aus einer Formulierung zur kontrollierten Freisetzung, umfassend:
a. Lamivudin oder ein pharmazeutisch verträgliches Derivat davon,
b. Zidovudin oder ein pharmazeutisch verträgliches Derivat davon und
c. ein Gemisch aus hydrophilen Polymeren, wobei die Polymere aus Celluloseethern, Polyuronsäuren und pharmazeutisch verträglichen Gummis oder Gemischen davon ausgewählt sind, und
d. ein pharmazeutisch verträgliches Calciumsalz.
ii. einer zweiten Schicht aus einer Formulierung zur sofortigen Freisetzung, wobei das NNRTI Nevirapin oder ein pharmazeutisch verträgliches Derivat davon ist, zusammen mit pharmazeutisch verträglichen Excipienten.

6. Antiretrovirales Arzneimittel nach Anspruch 1, umfassend eine selektive Kombination aus
i. einem Kern mit einer Formulierung zur kontrollierten Freisetzung, umfassend:
a. Lamivudin oder ein pharmazeutisch verträgliches Derivat davon,
b. Zidovudin oder ein pharmazeutisch verträgliches Derivat davon und
c. ein Gemisch aus hydrophilen Polymeren, wobei die Polymere aus Celluloseethern, Polyuronsäuren und pharmazeutisch verträglichen Gummis oder Gemischen davon ausgewählt sind, und
d. ein pharmazeutisch verträgliches Calciumsalz.
ii. einer äußeren Beschichtung aus einer Formulierung zur sofortigen Freisetzung, wobei das NNRTI Nevirapin oder ein pharmazeutisch verträgliches Derivat davon ist, und pharmazeutisch verträglichen Excipienten.

7. Antiretrovirales Arzneimittel nach Anspruch 1, umfassend eine selektive doppelschichtige Kombination aus
i. einer ersten Schicht aus einer Formulierung zur kontrollierten Freisetzung, umfassend:
a. Lamivudin oder ein pharmazeutisch verträgliches Derivat davon,
b. Zidovudin oder ein pharmazeutisch verträgliches Derivat davon und
c. ein Gemisch aus hydrophilen Polymeren, wobei die Polymere aus Celluloseethern, Polyuronsäuren und pharmazeutisch verträglichen Gummis oder Gemischen davon ausgewählt sind, und
d. ein pharmazeutisch verträgliches Calciumsalz.
ii. einer zweiten Schicht aus einer Formulierung zur sofortigen Freisetzung, wobei das NNRTI Efavirenz oder ein pharmazeutisch verträgliches Derivat davon ist, zusammen mit pharmazeutisch verträglichen Excipienten.

8. Antiretrovirales Arzneimittel nach Anspruch 1, umfassend eine selektive Kombination aus
i. einem Kern aus einer Formulierung zur kontrollierten Freisetzung, umfassend:
a. Lamivudin oder ein pharmazeutisch verträgliches Derivat davon,
b. Zidovudin oder ein pharmazeutisch verträgliches Derivat davon und
c. ein Gemisch aus hydrophilen Polymeren, wobei die Polymere aus Celluloseethern, Polyuronsäuren und pharmazeutisch verträglichen Gummis oder Gemischen davon ausgewählt sind, und
d. ein pharmazeutisch verträgliches Calciumsalz.
ii. einer äußeren Beschichtung aus einer Formulierung zur sofortigen Freisetzung, wobei das NNRTI Efavirenz oder ein pharmazeutisch verträgliches Derivat davon ist, zusammen mit pharmazeutisch verträglichen Excipienten.

9. Mittel nach einem der Ansprüche 1 bis 8, wobei die Menge an Lamivudin oder einem pharmazeutisch verträglichen Derivat davon 50 mg bis 500 mg beträgt.

10. Mittel nach Anspruch 9, wobei die Menge an Lamivudin oder einem pharmazeutisch verträglichen Derivat davon 300 mg beträgt.

11. Mittel nach einem der Ansprüche 1 bis 8, wobei die Menge an Zidovudin oder einem pharmazeutisch verträglichen Derivat davon 100 mg bis 1000 mg beträgt.

12. Mittel nach Anspruch 11, wobei die Menge an Zidovudin oder einem pharmazeutisch verträglichen Derivat davon 600 mg beträgt.

13. Mittel nach einem der Ansprüche 1 bis 12, wobei die Menge an Nevirapin/Efavirenz oder einem pharmazeutisch verträglichen Derivat davon 100 mg bis 1000 mg beträgt.

14. Mittel nach Anspruch 13, wobei die Menge an Nevirapin oder einem pharmazeutisch verträglichen Derivat davon 400 mg beträgt.

15. Mittel nach Anspruch 13, wobei die Menge an Efavirenz oder einem pharmazeutisch verträglichen Derivat davon 600 mg beträgt.

16. Mittel nach einem der Ansprüche 1 bis 15, wobei der Celluloseether aus Hydroxypropylcellulose, Hydoxypropylmethylcellulose, Carboxymethylcellulose, Natriumcarboxymethylcellulose, Ethylcellulose, Methylcellulose, Hydroxyethylcellulose und dergleichen ausgewählt ist.

17. Mittel nach Anspruch 16, wobei der Celluloseether Hydroxypropylmethylcellulose ist und in einer Menge von 2 Gew.-% bis 12 Gew.-% der Formulierung zur kontrollierten Freisetzung vorhanden ist.

18. Mittel nach Anspruch 17, wobei der Celluloseether Hydroxypropylmethylcellulose ist und in einer Menge von 3 Gew.-% bis 8 Gew.-% der Formulierung zur kontrollierten Freisetzung vorhanden ist.

19. Mittel nach einem der Ansprüche 1 bis 18, wobei die Polyuronsäure aus Alginsäure, Natriumalginat, Calciumalginat, Natriumcalciumalginat, Kaliumalginat, Ammoniumalginat, Magnesiumalginat und dergleichen ausgewählt ist.

20. Mittel nach Anspruch 19, wobei die Polyuronsäure Natriumalginat ist und in einer Menge von 0,5 Gew.-% bis 10 Gew.-% der Formulierung zur kontrollierten Freisetzung vorhanden ist.

21. Mittel nach Anspruch 20, wobei die Polyuronsäure Natriumalginat ist und in einer Menge von 1 Gew.-% bis 6 Gew.-% der Formulierung zur kontrollierten Freisetzung vorhanden ist.

22. Mittel nach einem der Ansprüche 1 bis 21, wobei der pharmazeutisch verträgliche Gummi aus Guargummi, Xanthangummi, Karayagummi, Tragantgummi, Akaziengummi und dergleichen ausgewählt ist.

23. Mittel nach Anspruch 22, wobei der pharmazeutisch verträgliche Gummi Guargummi ist und in einer Menge von 0,1 Gew.-% bis 10 Gew.-% der Formulierung zur kontrollierten Freisetzung vorhanden ist.

24. Mittel nach Anspruch 23, wobei der pharmazeutisch verträgliche Gummi Guargummi ist und in einer Menge von 0,5 Gew.-% bis 6 Gew.-% der Formulierung zur kontrollierten Freisetzung vorhanden ist.

25. Mittel nach einem der Ansprüche 1 bis 24, wobei das pharmazeutisch verträgliche Calciumsalz aus Calciumsulfat, Calciumphosphat, Calciumcarbonat und Calciumchlorid ausgewählt ist.

26. Mittel nach Anspruch 25, wobei das pharmazeutisch verträgliche Calciumsalz Calciumsulfat ist und in einer Menge von 0,1 Gew.-% bis 2,5 Gew.-% der Formulierung zur kontrollierten Freisetzung vorhanden ist.

27. Mittel nach Anspruch 26, wobei das pharmazeutisch verträgliche Calciumsalz Calciumsulfat ist und in einer Menge von 0,1 Gew.-% bis 2 Gew.-% der Formulierung zur kontrollierten Freisetzung vorhanden ist.

28. Mittel nach einem der Ansprüche 1 bis 27, wobei die Formulierung zur kontrollierten Freisetzung ferner mindestens ein in Wasser dispergierbares oder wasserlösliches Verdünnungsmittel umfasst, ausgewählt aus mikrokristalliner Cellulose, Dicalciumphosphat, Calciumcarbonat, Lactose, pulverisierter Cellulose, Stärke, Mannit und dergleichen.

29. Mittel nach Anspruch 28, wobei das Verdünnungsmittel mit 1 Gew.-% bis 28 Gew.-% der Formulierung zur kontrollierten Freisetzung vorhanden ist.

30. Mittel nach Anspruch 29, wobei das Verdünnungsmittel mikrokristalline Cellulose ist.

31. Mittel nach Anspruch 30, wobei die Menge an der mikrokristallinen Cellulose 5 Gew.-% bis 20 Gew.-% beträgt.

32. Mittel nach Anspruch 29, wobei das Verdünnungsmittel Dicalciumphosphat ist.

33. Mittel nach Anspruch 32, wobei die Menge an Dicalciumphosphat 1 Gew.-% bis 5 Gew.-% beträgt.

34. Mittel nach einem der Ansprüche 1 bis 33, wobei die Formulierung zur kontrollierten Freisetzung ferner mindestens ein Schmiermittel umfasst, ausgewählt aus Magnesiumstearat, Calciumstearat, Stearinsäure, Siliciumdioxid, Talkum und dergleichen.

35. Mittel nach Anspruch 34, wobei das Schmiermittel mit 0,1 Gew.-% bis 3 Gew.-% vorhanden ist.

36. Mittel nach einem der Ansprüche 1 bis 35, wobei die Formulierung zur sofortigen Freisetzung 10 Gew.-% bis 95 Gew.-% an Nevirapin oder einem pharmazeutisch verträglichen Derivat davon zusammen mit einem oder mehreren pharmazeutisch verträglichen Excipienten, ausgewählt aus Verdünnungsmitteln, Bindemitteln, Sprengmitteln, Schmiermitteln, Farbmitteln und dergleichen, umfasst.

37. Mittel nach Anspruch 36, wobei das Verdünnungsmittel aus mikrokristalliner Cellulose, Dicalciumphosphat, Calciumcarbonat, Lactose, pulverisierter Cellulose, Stärke, Mannit und dergleichen ausgewählt ist.

38. Mittel nach Anspruch 37, wobei das Verdünnungsmittel pulverisierte Cellulose ist und in einer Menge von 2 Gew.-% bis 15 Gew.-% der Formulierung zur sofortigen Freisetzung vorhanden ist.

39. Mittel nach Anspruch 36, wobei das Bindemittel aus Carboxymethylcellulose-Natrium, Povidone, vorgelatinisierter Stärke, Gelatine oder Gemischen davon ausgewählt ist.

40. Mittel nach Anspruch 39, wobei das Bindemittel in einer Menge von 1 Gew.-% bis 10 Gew.-% der Formulierung zur sofortigen Freisetzung vorhanden ist.

41. Mittel nach Anspruch 36, wobei das Sprengmittel aus Crospovidone, Natriumstärkeglycolat, vorgelatinisierter Stärke, Carboxymethylcellulose-Natrium, Croscarmellose-Natrium, Stärke und Gemischen davon ausgewählt ist.

42. Mittel nach Anspruch 41, wobei das Sprengmittel in einer Menge von 0,5 Gew.-% bis 15 Gew.-% der Formulierung zur sofortigen Freisetzung vorhanden ist.

43. Mittel nach Anspruch 36, wobei das Schmiermittel aus Magnesiumstearat, Calciumstearat, Stearinsäure, Siliciumdioxid, Talkum und dergleichen ausgewählt ist.

44. Mittel nach Anspruch 43, wobei das Schmiermittel mit 0,1 Gew.-% bis 3 Gew.-% vorhanden ist.

45. Verfahren zur Herstellung eines antiretroviralen Arzneimittels, umfassend (i) Bereitstellen einer Formulierung zur kontrollierten Freisetzung, indem Wirkstoffe, ausgewählt aus Lamivudin, Zidovudin oder Gemischen davon, mit hydrophilen Polymeren, ausgewählt aus Celluloseethern, Polyuronsäuren, pharmazeutisch verträglichen Gummis oder Gemischen davon, und mit einem pharmazeutisch verträglichen Calciumsalz, gegebenenfalls einem Verdünnungsmittel und einem Schmiermittel, zusammen gemischt werden, (ii) Vermischen einer Formulierung zur sofortigen Freisetzung, die aus mindestens dem NNRTI erhalten wird, mit pharmazeutisch verträglichen Excipienten und (iii) Erhalten des Mittels daraus durch Komprimieren der resultierenden Mischungen zu doppelschichtigen Tabletten.

46. Verfahren zur Herstellung eines antiretroviralen Arzneimittels, umfassend (i) Bereitstellen eines Kerns aus der Formulierung zur kontrollierten Freisetzung, indem Wirkstoffe, ausgewählt aus Lamivudin, Zidovudin oder Gemischen davon, mit hydrophilen Polymeren, ausgewählt aus Celluloseethern, Polyuronsäuren, pharmazeutisch verträglichen Gummis oder Gemischen davon, und mit einem pharmazeutisch verträglichen Calciumsalz, gegebenenfalls einem Verdünnungsmittel und einem Schmiermittel, zusammen gemischt werden, (ii) Bereitstellen einer äußeren Beschichtung aus einer Formulierung zur sofortigen Freisetzung, die aus mindestens dem NNRTI, ausgewählt aus Nevirapin und Efavirenz, erhalten wird, das mit pharmazeutisch verträglichen Excipienten vermischt ist, und (iii) Erhalten des Mittels daraus durch Auftragen der äußeren Beschichtung über dem Kern durch Komprimierbeschichtung.

47. Verfahren nach Anspruch 45 oder 46, wobei jede Mischung vor dem Komprimieren trocken granuliert wird.

48. Verfahren nach Anspruch 45 oder 46, wobei jede Mischung vor dem Komprimieren feucht granuliert wird.

49. Verwendung des antiretroviralen Arzneimittels aus drei Arzneistoffen, umfassend eine Kombination aus Lamivudin und Zidovudin als eine Komponente zur kontrollierten Freisetzung und Nevirapin oder Efavirenz als eine Komponente zur sofortigen Freisetzung, zur Herstellung eines Medikaments zur Verringerung der Pillenlast bei einem Patienten, der an einer HIV-Infektion und/oder erworbenem Immundefektsyndrom (AIDS) leidet.

50. Verwendung des antiretroviralen Arzneimittels aus drei Arzneistoffen, umfassend eine Kombination aus Lamivudin und Zidovudin als eine Komponente zur kontrollierten Freisetzung und Nevirapin oder Efavirenz als eine Komponente zur sofortigen Freisetzung, zur Herstellung eines Medikaments zur Verringerung der Pillenlast bei einem Patienten, der an einer HIV-Infektion und/oder erworbenem Immundefektsyndrom (AIDS) leidet, so dass das Mittel bei der Verabreichung an den Patienten eine Cₘₐₓ von etwa 1,2 bis 2,0 µg/ml für Lamivudin, etwa 1,0 bis 2,0 µg/ml für Zidovudin und etwa 4,5 bis 5,5 µg/ml für Nevirapin bereitstellt.

51. Verwendung des antiretroviralen Arzneimittels aus drei Arzneistoffen, umfassend eine Kombination aus Lamivudin und Zidovudin als eine Komponente zur kontrollierten Freisetzung und Nevirapin oder Efavirenz als eine Komponente zur sofortigen Freisetzung, zur Herstellung eines Medikaments zur Verringerung der Pillenlast bei einem Patienten, der an einer HIV-Infektion und/oder erworbenem Immundefektsyndrom (AIDS) leidet, so dass das Mittel bei der Verabreichung an den Patienten eine AUC₀₋ₜ von etwa 8 bis 14 µg·h/ml für Lamivudin, etwa 5 bis 9 µg·h/ml für Zidovudin und etwa 32 bis 40 µg·h/ml für Nevirapin bereitstellt.

52. Verwendung des antiretroviralen Arzneimittels aus drei Arzneistoffen, umfassend eine Kombination aus Lamivudin und Zidovudin als eine Komponente zur kontrollierten Freisetzung und Nevirapin oder Efavirenz als eine Komponente zur sofortigen Freisetzung, zur Herstellung eines Medikaments zur Erhöhung der *in*-*vivo*-Halbwertszeit von Lamivudin und Zidovudin, während die Halbwertszeit von Nevirapin nicht beeinflusst wird, und somit zur Verringerung der Pillenlast bei einem Patienten, der an einer HIV-Infektion und/oder erworbenem Immundefektsyndrom (AIDS) leidet.

53. Verwendung nach Anspruch 51, wobei das Mittel bei der Verabreichung an den Patienten eine t_{1/2} von etwa 5 h für Lamivudin, etwa 2 h für Zidovudin und etwa 29 h für Nevirapin bereitstellt.

54. Verwendung des antiretroviralen Arzneimittels aus drei Arzneistoffen, umfassend eine Kombination aus Lamivudin und Zidovudin als eine Komponente zur kontrollierten Freisetzung und Nevirapin oder Efavirenz als eine Komponente zur sofortigen Freisetzung, zur Herstellung eines Medikaments zur Verringerung der Pillenlast bei einem Patienten, der an einer HIV-Infektion und/oder erworbenem Immundefektsyndrom (AIDS) leidet, so dass das Mittel bei der Verabreichung an den Patienten eine Cₘₐₓ der Arzneistoffe in der Komponente zur kontrollierten Freisetzung bereitstellt, die im Wesentlichen gleich derjenigen ist, die von Zusammensetzungen zur sofortigen Freisetzung der Arzneistoffe bereitgestellt wird, wenn sie simultan, nacheinander oder gleichzeitig eingenommen werden.

## Revendications

1. Composition pharmaceutique antirétrovirale comprenant une combinaison sélective de :
i. une formulation à libération contrôlée comprenant :
a. de la lamivudine ou un dérivé pharmaceutiquement acceptable de celle-ci,
b. de la zidovudine ou un dérivé pharmaceutiquement acceptable de celle-ci, et
c. un mélange de polymères hydrophiles, lesdits polymères étant choisis dans le groupe constitué par les éthers de cellulose, les acides polyuroniques et les gommes pharmaceutiquement acceptables ou des mélanges de ceux-ci et
d. un sel de calcium pharmaceutiquement acceptable,
ii. une formulation à libération immédiate comprenant au moins un médicament inhibiteur non nucléosidique de la transcriptase inverse (INNTI) sélectif ou un déviré pharmaceutiquement acceptable de celui-ci conjointement avec des excipients pharmaceutiquement acceptables.

2. Composition pharmaceutique antirétrovirale selon la revendication 1, dans laquelle ledit médicament INNTI est choisi parmi la névirapine et l'éfavirenz.

3. Composition pharmaceutique antirétrovirale selon la revendication 1, qui est sous la forme de deux couches séparées de ladite formulation à libération contrôlée et de la formulation à libération immédiate.

4. Composition pharmaceutique antirétrovirale selon la revendication 1, qui est sous la forme d'un coeur de ladite formulation à libération contrôlée et d'un enrobage externe de la formulation à libération immédiate.

5. Composition pharmaceutique antirétrovirale selon la revendication 1, comprenant une combinaison bicouche sélective de :
i. une première couche de formulation à libération contrôlée comprenant:
a. de la lamivudine ou un dérivé pharmaceutiquement acceptable de celle-ci,
b. de la zidovudine ou un dérivé pharmaceutiquement acceptable de celle-ci, et
c. un mélange de polymères hydrophiles, lesdits polymères étant choisis dans le groupe constitué par les éthers de cellulose, les acides polyuroniques et les gommes pharmaceutiquement acceptables ou les mélanges de ceux-ci et
d. un sel de calcium pharmaceutiquement acceptable,
ii. une seconde couche d'une formulation à libération immédiate dans laquelle ledit INNTI est la névirapine ou un dérivé pharmaceutiquement acceptable de celle-ci conjointement avec des excipients pharmaceutiquement acceptables.

6. Composition pharmaceutique antirétrovirale selon la revendication 1, comprenant une combinaison sélective de :
i. un coeur ayant une formulation à libération contrôlée comprenant :
a. de la lamivudine ou un dérivé pharmaceutiquement acceptable de celle-ci,
b. de la zidovudine ou un dérivé pharmaceutiquement acceptable de celle-ci, et
c. un mélange de polymères hydrophiles, lesdits polymères étant choisis dans le groupe constitué par les éthers de cellulose, les acides polyuroniques et les gommes pharmaceutiquement acceptables ou les mélanges de ceux-ci et
d. un sel de calcium pharmaceutiquement acceptable,
ii. un enrobage externe d'une formulation à libération immédiate dans laquelle ledit INNTI est la névirapine ou un dérivé pharmaceutiquement acceptable de celle-ci et des excipients pharmaceutiquement acceptables.

7. Composition pharmaceutique antirétrovirale selon la revendication 1, comprenant une combinaison bicouche sélective de :
i. une première couche d'une formulation à libération contrôlée comprenant :
a. de la lamivudine ou un dérivé pharmaceutiquement acceptable de celle-ci,
b. de la zidovudine ou un dérivé pharmaceutiquement acceptable de celle-ci, et
c. un mélange de polymères hydrophiles, lesdits polymères étant choisis dans le groupe constitué par les éthers de cellulose, les acides polyuroniques et les gommes pharmaceutiquement acceptables ou les mélanges de ceux-ci et
d. un sel de calcium pharmaceutiquement acceptable,
ii. une seconde couche d'une formulation à libération immédiate dans laquelle ledit INNTI est l'éfavirenz ou un dérivé pharmaceutiquement acceptable de celui-ci conjointement avec des excipients pharmaceutiquement acceptables.

8. Composition pharmaceutique antirétrovirale selon la revendication 1, comprenant une combinaison sélective de :
i. un coeur de formulation à libération contrôlée comprenant:
a. de la lamivudine ou un dérivé pharmaceutiquement acceptable de celle-ci,
b. de la zidovudine ou un dérivé pharmaceutiquement acceptable de celle-ci, et
c. un mélange de polymères hydrophiles, lesdits polymères étant choisis dans le groupe constitué par les éthers de cellulose, les acides polyuroniques et les gommes pharmaceutiquement acceptables ou les mélanges de ceux-ci et
d. un sel de calcium pharmaceutiquement acceptable,
ii. un enrobage externe d'une formulation à libération immédiate dans laquelle ledit INNTI est l'éfavirenz ou un dérivé pharmaceutiquement acceptable de celui-ci conjointement avec des excipients pharmaceutiquement acceptables.

9. Composition selon l'une quelconque des revendications 1 à 8, dans laquelle la quantité de lamivudine ou de dérivé pharmaceutiquement acceptable de celle-ci est de 50 mg à 500 mg.

10. Composition selon la revendication 9, dans laquelle la quantité de lamivudine ou de dérivé pharmaceutiquement acceptable de celle-ci est de 300 mg.

11. Composition selon l'une quelconque des revendications 1 à 8, dans laquelle la quantité de zidovudine ou de dérivé pharmaceutiquement acceptable de celle-ci est de 100 mg à 1 000 mg.

12. Composition selon la revendication 11, dans laquelle la quantité de zidovudine ou de dérivé pharmaceutiquement acceptable de celle-ci est de 600 mg.

13. Composition selon l'une quelconque des revendications 1 à 12, dans laquelle la quantité de névirapine/éfavirenz ou de dérivé pharmaceutiquement acceptable de ceux-ci est de 100 mg à 1 000 mg.

14. Composition selon la revendication 13, dans laquelle la quantité de névirapine ou de dérivé pharmaceutiquement acceptable de celle-ci est de 400 mg.

15. Composition selon la revendication 13, dans laquelle la quantité d'éfavirenz ou de dérivé pharmaceutiquement acceptable de celui-ci est de 600 mg.

16. Composition selon l'une quelconque des.revendications 1 à 15, dans laquelle l'éther de cellulose est choisi parmi l'hydroxypropylcellulose, l'hydroxypropylméthylcellulose, la carboxyméthylcellulose, la carboxyméthylcellulose sodique, l'éthylcellulose, la méthylcellulose, l'hydroxy éthylcellulose et équivalents.

17. Composition selon la revendication 16, dans laquelle l'éther de cellulose est l'hydroxypropylméthylcellulose et est présent en une quantité de 2 % à 12 % en poids de la formulation à libération contrôlée.

18. Composition selon la revendication 17, dans laquelle l'éther de cellulose est l'hydroxypropylméthylcellulose et est présent en une quantité de 3 % à 8 % en poids de la formulation à libération contrôlée.

19. Composition selon l'une quelconque des revendications 1 à 18, dans laquelle l'acide polyuronique est choisi parmi l'acide alginique, l'alginate de sodium, l'alginate de calcium, l'alginate de calcium et de sodium, l'alginate de potassium, l'alginate d'ammonium, l'alginate de magnésium et équivalents.

20. Composition selon la revendication 19, dans laquelle l'acide polyuronique est l'alginate de sodium et est présent en une quantité de 0,5 % à 10 % en poids de la formulation à libération contrôlée.

21. Composition selon la revendication 20, dans laquelle l'acide polyuronique est l'alginate de sodium et est présent en une quantité de 1 % à 6 % en poids de la formulation à libération contrôlée.

22. Composition selon l'une quelconque des revendications 1 à 21, dans laquelle la gomme pharmaceutiquement acceptable est choisie parmi la gomme de guar, la gomme de Xanthane, la gomme de karaya, la gomme de tragacanthe, la gomme d'acacia et équivalents.

23. Composition selon la revendication 22, dans laquelle la gomme pharmaceutiquement acceptable est la gomme de guar et est présente en une quantité de 0,1 % à 10 % en poids de la formulation à libération contrôlée.

24. Composition selon la revendication 23, dans laquelle la gomme pharmaceutiquement acceptable est la gomme de guar et est présente en une quantité de 0,5 % à 6 % en poids de la formulation à libération contrôlée.

25. Composition selon l'une quelconque des revendications 1 à 24, dans laquelle le sel de calcium pharmaceutiquement acceptable est choisi dans le groupe constitué par le sulfate de calcium, le phosphate de calcium, le carbonate de calcium et le chlorure de calcium.

26. Composition selon la revendication 25, dans laquelle le sel de calcium pharmaceutiquement acceptable est le sulfate de calcium et est présent en une quantité de 0,1 % à 2,5 % en poids de la formulation à libération contrôlée.

27. Composition selon la revendication 26, dans laquelle le sel de calcium pharmaceutiquement acceptable est le sulfate de calcium et est présent en une quantité de 0,1 % à 2 % en poids de la formulation à libération contrôlée.

28. Composition selon l'une quelconque des revendications 1 à 27, dans laquelle la formulation à libération contrôlée comprend en outre au moins un diluant dispersible dans l'eau ou soluble dans l'eau choisi parmi la cellulose microcristalline, le phosphate dicalcique, le carbonate de calcium, le lactose, la cellulose pulvérulente, l'amidon, le mannitol, et équivalents.

29. Composition selon la revendication 28, dans laquelle le diluant est présent en une quantité de 1 % à 28 % en poids de la formulation à libération contrôlée.

30. Composition selon la revendication 29, dans laquelle le diluant est la cellulose microcristalline.

31. Composition selon la revendication 30, dans laquelle la quantité de cellulose microcristalline est de 5 % à 20 % en poids.

32. Composition selon la revendication 29, dans laquelle le diluant est le phosphate dicalcique.

33. Composition selon la revendication 32, dans laquelle la quantité de phosphate dicalcique est de 1 % à 5 % en poids.

34. Composition selon l'une quelconque des revendications 1 à 33, dans laquelle la formulation à libération contrôlée comprend en outre au moins un lubrifiant choisi parmi le stéarate de magnésium, le stéarate de calcium, l'acide stéarique, le dioxyde de silicium, le talc et équivalents.

35. Composition selon la revendication 34, dans laquelle le lubrifiant est présent en une quantité de 0,1 % à 3 % en poids.

36. Composition selon l'une quelconque des revendications 1 à 35, dans laquelle la formulation à libération immédiate comprend de 10 % à 95 % en poids de névirapine ou d'un dérivé pharmaceutiquement acceptable de celle-ci conjointement avec un ou plusieurs excipients pharmaceutiquement acceptables choisis parmi des diluants, des liants, des agents de désagrégation, des lubrifiants, des agents colorants et équivalents.

37. Composition selon la revendication 36, dans laquelle le diluant est choisi parmi la cellulose microcristalline, le phosphate dicalcique, le carbonate de calcium, le lactose, la cellulose pulvérulente, l'amidon, le mannitol et équivalents.

38. Composition selon la revendication 37, dans laquelle le diluant est la cellulose pulvérulente et est présent en une quantité de 2 % à 15 % en poids de la formulation à libération immédiate.

39. Composition selon la revendication 36, dans laquelle le liant est choisi parmi la carboxyméthylcellulose sodique, la povidone, l'amidon prégélatinisé, la gélatine ou des mélanges de ceux-ci.

40. Composition selon la revendication 39, dans laquelle le liant est présent en une quantité de 1 % à 10 % en poids de la formulation à libération immédiate.

41. Composition selon la revendication 36, dans laquelle l'agent de désagrégation est choisi parmi la crospovidone, le glycolate d'amidon sodique, l'amidon prégélatinisé, la carboxyméthylcellulose sodique, la croscarmellose sodique, l'amidon et des mélanges de ceux-ci.

42. Composition selon la revendication 41, dans laquelle l'agent de désagrégation est présent en une quantité de 0,5 % à 15 % en poids de la formulation à libération immédiate.

43. Composition selon la revendication 36, dans laquelle le lubrifiant est choisi parmi le stéarate de magnésium, le stéarate de calcium, l'acide stéarique, le dioxyde de silicium, le talc et équivalents.

44. Composition selon la revendication 43, dans laquelle le lubrifiant est présent en une quantité de 0,1 % à 3 % en poids.

45. Processus de préparation d'une composition pharmaceutique antirétrovirale comprenant (i) la fourniture d'une formulation à libération contrôlée en mélangeant ensemble les ingrédients actifs choisis parmi la lamivudine, la zidovudine ou les mélanges de celles-ci avec des polymères hydrophiles choisis parmi les éthers de cellulose, les acides polyuroniques, les gommes pharmaceutiquement acceptables ou des mélanges de ceux-ci, et avec un sel de calcium pharmaceutiquement acceptable, facultativement un diluant et un lubrifiant, (ii) le mélange d'une formulation à libération immédiate obtenue du mélange du au moins un NNRTI avec des excipients pharmaceutiquement acceptables et (iii) l'obtention de la composition à partir de ceci par compression des mélanges résultants en des comprimés bicouches.

46. Processus de préparation d'une composition pharmaceutique antirétrovirale comprenant (i) la fourniture d'un coeur de ladite formulation à libération contrôlée en mélangeant ensemble les ingrédients actifs choisis parmi la lamivudine, la zidovudine ou les mélanges de celles-ci avec des polymères hydrophiles choisis parmi les éthers de cellulose, les acides polyuroniques, les gommes pharmaceutiquement acceptables ou les mélanges de ceux-ci, et avec un sel de calcium pharmaceutiquement acceptable, facultativement un diluant et un lubrifiant, (ii) la fourniture d'un enrobage externe d'une formulation à libération immédiate obtenue du au moins un NNRTI choisi parmi la névirapine et l'éfavirenz mélangés avec des excipients pharmaceutiquement acceptables et (iii) l'obtention de la composition à partir de ceci par application dudit enrobage externe sur ledit coeur par enrobage par compression.

47. Processus selon la revendication 45 ou 46, dans lequel chaque mélange est granulé par voie sèche avant la compression.

48. Processus selon la revendication 45 ou 46, dans lequel chaque mélange est granulé par voie humide avant la compression.

49. Utilisation d'une composition pharmaceutique antirétrovirale à trois médicaments comprenant une combinaison de lamivudine et de zidovudine en tant qu'un composant à libération contrôlée et de la névirapine ou de l'éfavirenz en tant qu'un composant à libération immédiate pour la préparation d'un médicament destiné à réduire la prise de médicaments chez un patient souffrant d'une infection par le VIH et/ou du syndrome d'immunodéficience acquise.

50. Utilisation d'une composition pharmaceutique antirétrovirale à trois médicaments comprenant une combinaison de lamivudine et de zidovudine en tant qu'un composant à libération contrôlée et de la névirapine ou de l'éfavirenz en tant qu'un composant à libération immédiate pour la préparation d'un médicament destiné à réduire la prise de médicaments chez un patient souffrant d'une infection par le VIH et/ou du syndrome d'immunodéficience acquise de telle manière que la composition, lors de l'administration au dit patient, fournit une Cₘₐₓ d'environ 1,2 à 2,0 µg/mL pour la lamivudine, d'environ 1,0 à 2,0 µg/mL pour la zidovudine et d'environ 4,5 à 5,5 µg/mL pour la névirapine.

51. Utilisation d'une composition pharmaceutique antirétrovirale à trois médicaments comprenant une combinaison de lamivudine et de zidovudine en tant qu'un composant à libération contrôlée et de la névirapine ou de l'éfavirenz en tant qu'un composant à libération immédiate pour la préparation d'un médicament destiné à réduire la prise de médicaments chez un patient souffrant d'une infection par le VIH et/ou du syndrome d'immunodéficience acquise de telle manière que la composition, lors de l'administration au dit patient, fournit une AUC₀₋ₜ d'environ 8 à 14 µg.hr/mL pour la lamivudine, d'environ 5 à 9 µg.hr/mL pour la zidovudine et d'environ 32 à 40 µg.hr/mL pour la névirapine.

52. Utilisation d'une composition pharmaceutique antirétrovirale à trois médicaments comprenant une combinaison de lamivudine et de zidovudine en tant qu'un composant à libération contrôlée et de névirapine ou d'éfavirenz en tant qu'un composant à libération immédiate pour la préparation d'un médicament destiné à faire augmenter la demi-vie in vivo de lamivudine et de zidovudine sans affecter la demi-vie de la névirapine et ainsi réduire la prise de médicaments chez un patient souffrant d'une infection par le VIH et/ou du syndrome d'immunodéficience acquise.

53. Utilisation selon la revendication 51, dans laquelle ladite composition, lors de l'administration au dit patient, fournit une t_{1/2} d'environ 5 heures pour la lamivudine, d'environ 2 heures pour la zidovudine et d'environ 29 heures pour la névirapine.

54. Utilisation d'une composition pharmaceutique antirétrovirale à trois médicaments comprenant une combinaison de lamivudine et de zidovudine en tant qu'un composant à libération contrôlée et de la névirapine ou de l'éfavirenz en tant qu'un composant à libération immédiate pour la préparation d'un médicament destiné à réduire la prise de médicaments chez un patient souffrant d'une infection par le VIH et/ou du syndrome d'immunodéficience acquise de telle manière que la composition, lors de l'administration au dit patient, fournit une Cₘₐₓ desdits médicaments dans le composant à libération contrôlée qui est substantiellement similaire à celle fournie par les compositions à libération immédiate desdits médicaments lorsqu'ils sont pris de manière simultanée, séquentielle ou concurrente.
